# EUROPEAN PATENT APPLICATION

(11) **EP 4 792 847 A2**
(43) Date of publication of application: **19.08.2026**
(21) Application number: 26159449.3
(22) Date of filing: 18.02.2026
(51) Int. Cl.: A61M 1/14, A61M 1/34, A61M 1/36

(54) **PANEL KIT FOR BLOOD PURIFICATION DEVICE**

(30) Priority: 18.02.2025 CN 202510183366; 04.02.2026 CN 202610163324
(71) Applicant: Shenzhen Mindray Bio-Medical Electronics Co., Ltd., Shenzhen, Guangdong 518057 (CN)
(72) Inventor: LUO, Caijin, Shenzhen, 518057 (CN); ZOU, Zhihua, Shenzhen, 518057 (CN); WEI, Kai, Shenzhen, 518057 (CN); ZOU, Huan, Shenzhen, 518057 (CN); AI, Shiming, Shenzhen, 518057 (CN)
(74) Representative: KIPA AB

(57) **Abstract**

Disclosed is a panel kit for a blood purification device, including a power panel, a monitoring panel and a pipeline assembly. The power panel and the monitoring panel can be detachably mounted on a device housing of the blood purification device separately. The pipeline assembly is configured to connect a filter to a blood vessel of a patient and a medical liquid container respectively, and includes at least two pump pipeline sections mounted on the power panel, and when being adapted to a pump assembly of the blood purification device, the pump pipeline sections can be driven by the pump assembly to drive a blood or a medical liquid to flow. The pipeline assembly further includes at least two monitoring pipeline sections mounted on the monitoring panel. At least one monitoring pipeline section is fixedly provided with a first interface.

## Description

### CROSS-REFERENCE TO RELATED APPLICATION

The present disclosure claims priority of Chinese Patent Application No. 202510183366.5, entitled " PANEL KIT FOR BLOOD PURIFICATION DEVICE", filed with the China National Intellectual Property Administration on February 18, 2025, and Chinese Patent Application No. 202610163324.X, entitled " PANEL KIT FOR BLOOD PURIFICATION DEVICE", filed with the China National Intellectual Property Administration on February 4, 2026.

### TECHNICAL FIELD

The present disclosure belongs to the field of medical devices, and in particular, relates to a panel kit for a blood purification device.

### BACKGROUND

Blood purification therapy is a treatment method that draws a patient's blood out of the body and then removes pathogenic substances from the patient's blood by using a filter element to achieve blood purification. The main treatment methods include hemodialysis, hemofiltration, hemodiafiltration, hemoperfusion, plasma exchange and the like.

In the related art, blood purification therapy is usually implemented by connecting a blood purification device to a blood vessel of the patient. Therefore, a large number of pipeline sections and the like need to be arranged on the blood purification device to deliver the patient's blood, replacement fluid, anticoagulant and the like. In addition, to ensure the safety of the blood returned to the patient's body, the pipeline sections need to be replaced after each use, and the large number of pipeline sections results in high difficulty in their mounting.

### SUMMARY

In a first aspect, an embodiment of the present disclosure provides a panel kit for a blood purification device, comprising:
a power panel and a monitoring panel, wherein the power panel and the monitoring panel are independent of each other and are configured to be detachably mounted on a device housing of the blood purification device separately; and
a pipeline assembly configured to connect a filter to a blood vessel of a patient and a medical liquid container respectively; wherein
the pipeline assembly includes at least two pump pipeline sections mounted on the power panel, and if the pump pipeline sections are adapted to a pump assembly of the blood purification device, the pump pipeline sections are configured to be driven by the pump assembly, so as to drive a blood or a medical liquid to flow;
the pipeline assembly further includes at least two monitoring pipeline sections mounted on the monitoring panel, the monitoring pipeline sections are configured to monitor parameter information of the blood and/or the medical liquid, the parameter information at least includes a pressure parameter, at least one of the monitoring pipeline sections is provided with a first interface, and if the first interface is connected to a second interface of the blood purification device, the monitoring pipeline section is used for the blood purification device to monitor the parameter information; and the first interface is fixedly arranged relative to the monitoring panel during a process of mounting the monitoring panel on the device housing.

In a second aspect, an embodiment of the present disclosure further provides a panel kit for a blood purification device, comprising:
a power panel and a monitoring panel, wherein the power panel and the monitoring panel are independent of each other and are configured to be detachably mounted on a device housing of the blood purification device separately; and
a pipeline assembly configured to connect a filter to a blood vessel of a patient and a medical liquid container respectively; wherein
the pipeline assembly includes at least two pump pipeline sections mounted on the power panel, and if the pump pipeline sections are adapted to a pump assembly of the blood purification device, the pump pipeline sections are configured to be driven by the pump assembly, so as to drive a blood or a medical liquid to flow;
the pipeline assembly further includes at least two monitoring pipeline sections mounted on the monitoring panel, the monitoring pipeline sections are configured to monitor parameter information of the blood and/or the medical liquid, the parameter information at least includes a pressure parameter, at least one of the monitoring pipeline sections is provided with a first interface, and if the first interface is connected to a second interface of the blood purification device, the monitoring pipeline section is used for the blood purification device to monitor the parameter information; and the first interface is movably arranged relative to the monitoring panel and the movable arrangement allows a user to operate the first interface, so as to achieve the connection between the first interface and the second interface.

In the embodiments of the present disclosure, on the one hand, by arranging at least two pump pipeline sections on the power panel, a user can be assisted in the mounting of the at least two pump pipeline sections through the mounting operation of the power panel, thereby reducing the mounting difficulty and the probability of mismounting of the at least two pump pipeline sections; on the other hand, by arranging at least two monitoring pipeline sections on the monitoring panel, the user can be assisted in the mounting of the at least two monitoring pipeline sections through the mounting operation of the monitoring panel, thereby reducing the mounting difficulty and the probability of mismounting of the at least two monitoring pipeline sections. In addition, during mounting of the monitoring panel on the device housing, when the first interface is fixedly arranged relative to the monitoring panel, the first interface can be mounted in place correspondingly after the monitoring panel is mounted in place, thus saving the subsequent step of manually assembling the first interface with the second interface; when the first interface is movably arranged relative to the monitoring panel, the user can conveniently manually connect the first interface with the second interface after the monitoring panel is mounted in place, so as to avoid the difficulty in mounting the first interface in place at one time, thereby reducing the mounting difficulty of the panel kit. In view of the above, the panel kit according to the embodiments of the present disclosure has the advantages of easy mounting and high accuracy of detection results of the parameter components.

### BRIEF DESCRIPTION OF THE DRAWINGS

The technical solutions and beneficial effects of the present disclosure will become apparent from the detailed description of the specific embodiments of the present disclosure in conjunction with the accompanying drawings.
Fig. 1 is a structural schematic diagram of a blood purification device according to an embodiment of the present disclosure.
Fig. 2 is a structural schematic diagram of fitting and mounting of the blood purification device and the panel kit shown in Fig. 1.
Fig. 3 is a second structural schematic diagram of a power assembly of the panel kit shown in Fig. 2.
Fig. 4 is a second structural schematic diagram of a monitoring panel of the panel kit shown in Fig. 2.
Fig. 5 is a structural schematic diagram of a monitoring pipeline section of the panel kit shown in Fig. 2.
Fig. 6 is a structural schematic diagram of a secondary membrane pressure monitoring pipeline section of the panel kit in an embodiment of the present disclosure.
Fig. 7 is a schematic diagram of a flow path of medical liquid flowing out of a filter in the panel kit shown in Fig. 2.
Fig. 8 is a schematic diagram of a blood flow path in the panel kit shown in Fig. 2.
Fig. 9 is a schematic diagram of a flow path of an anticoagulant in the panel kit shown in Fig. 2.
Fig. 10 is a schematic diagram of flow paths of replacement fluid and dialysate in the panel kit shown in Fig. 2.
Fig. 11 is a second structural schematic diagram of the panel kit in an embodiment of the present disclosure.
Fig. 12 is a third structural schematic diagram of the monitoring panel of the panel kit shown in Fig. 2.

The reference numerals in the figures are respectively:
100 - panel kit;
11 - power panel; 111 - first connecting part; 112 - first region; 113 - second region;12 - monitoring panel; 121 - second connecting part;
13 - pipeline assembly; 131 - pump pipeline section; 1311 - waste liquid pressure pump pipeline section; 1312 - blood pump pipeline section; 1313 - dialysate pump pipeline section; 1314 - replacement fluid pump pipeline section; 1315 - front pump pipeline section before blood pump; 1316 - medical liquid pump pipeline section; 132 - monitoring pipeline section; 1321 - first interface; 1322 - blood leakage monitoring pipeline section; 1323 - secondary membrane pressure monitoring pipeline section; 1324 - waste liquid pressure monitoring pipeline section; 1325 - blood drawing pressure monitoring pipeline section; 1326 - pressure monitoring pipeline section before filtering; 133 - pressure monitoring module; 1331 - fluid part; 1332 - gas part; 1333 - diaphragm; 13401 - connecting flow path; 13402 - waste liquid flow path; 13403 - collection flow path; 13404 - blood drawing flow path; 13405 - blood pumping flow path; 13406 - blood return flow path; 13407 - injection flow path; 13408 - first liquid inlet flow path; 13409 - second liquid inlet flow path; 13410 - third liquid inlet flow path; 13411 - fourth liquid inlet flow path; 13412 - fifth liquid inlet flow path; 13413 - sixth liquid inlet flow path; 13414 - heating container; 13415 - liquid outlet flow path; 13416 - first liquid outlet flow path; 13417 - second liquid outlet flow path; 13418 - third liquid outlet flow path; 13419 - degassing pot; 13420 - degassing pot monitoring element; 13421 - first heating container; 13422 - second heating container; 135 - electrostatic discharge element;
14 - heating panel;
200 - blood purification device;
21 - device housing; 22 - pump assembly; 23 - second interface; 24 - first driving mechanism; 241 - first claw; 25 - first position detection mechanism; 26 - second position detection mechanism; 27 - second driving mechanism; 271 - second claw; 28 - first pipeline switching device; 29 - second pipeline switching device; and
300 - filter.

### DETAILED DESCRIPTION

The technical solutions in the embodiments of the present disclosure will be clearly and completely described below in conjunction with the accompanying drawings in the embodiments of the present disclosure. Obviously, the described embodiments are only a part of the embodiments of the present disclosure, rather than all the embodiments. All other embodiments obtained by those skilled in the art without creative work based on the embodiments in the present disclosure fall within the protection scope of the present disclosure.

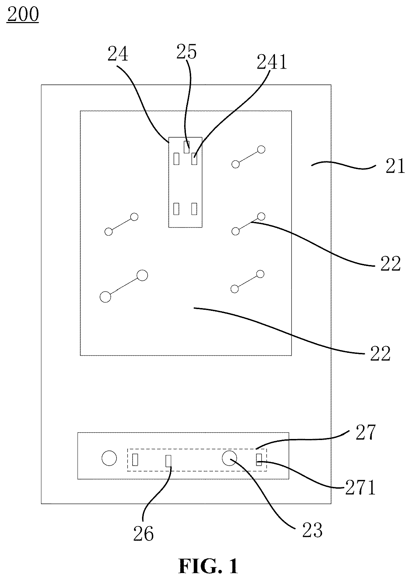

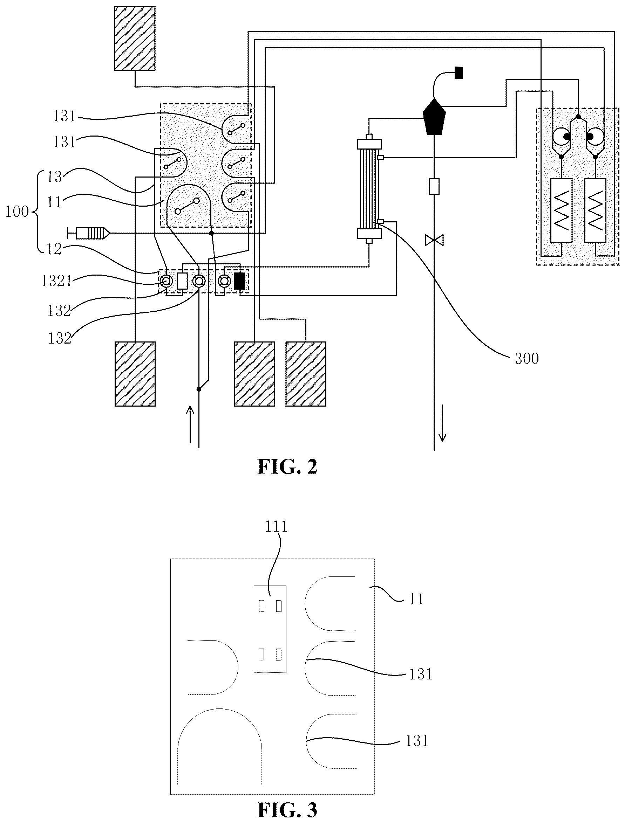

Referring to Figs. 1 and 2, an embodiment of the present disclosure provides a panel kit 100 for a blood purification device, the panel kit 100 includes a power panel 11, a monitoring panel 12 and a pipeline assembly 13. The power panel 11 and the monitoring panel 12 are configured to be detachably mounted on a device housing 21 of a blood purification device 200 separately. The pipeline assembly 13 is configured to connect a filter 300 to a blood vessel of a patient and a medical liquid container respectively.

It should be noted here that in the embodiment of the present disclosure, the power panel 11 and the monitoring panel 12 can be detached and mounted separately, which means that the power panel 11 and the monitoring panel 12 are independent of each other and can be detached and mounted separately.

Here, the pipeline assembly 13 includes at least two pump pipeline sections 131 mounted on the power panel 11, and if the pump pipeline sections 131 are adapted to a pump assembly 22 of the blood purification device 200, the pump pipeline sections 131 are configured to be driven by the pump assembly 22, so as to drive a blood or a medical liquid to flow. Therefore, by arranging the at least two pump pipeline sections 131 on the power panel 11, a user's mounting operation of the power panel 11 can be helpful to the mounting of the at least two pump pipeline sections 131, thereby reducing the mounting difficulty and the probability of mismounting of the at least two pump pipeline sections 131.

The pipeline assembly 13 further includes at least two monitoring pipeline sections 132 mounted on the monitoring panel 12, the monitoring pipeline sections 132 are configured to monitor parameter information of the blood and/or the medical liquid, and the parameter information at least includes a pressure parameter. Thus, by arranging the at least two monitoring pipeline sections 132 on the monitoring panel 12, the mounting operation of the monitoring panel 12 can be helpful to the mounting of the at least two pump pipeline sections 131, thereby reducing the mounting difficulty and the probability of mismounting of the at least two pump pipeline sections 131.

At least one of the monitoring pipeline sections 132 is provided with a first interface 1321, and if the first interface 1321 is connected to a second interface 23 of the blood purification device 200, the monitoring pipeline section 132 is used for the blood purification device 200 to monitor the parameter information.

Here, during a process of mounting the monitoring panel 12 on the device housing 21, the first interface 1321 is either fixedly arranged relative to the monitoring panel 12 or movably arranged relative to the monitoring panel 12.

When the first interface 1321 is fixedly arranged relative to the monitoring panel 12, since the first interface 1321 is fixedly arranged relative to the monitoring panel 12 during the mounting of the monitoring panel 12 on the device housing 21, after the monitoring panel 12 is mounted in place, the first interface 1321 can be mounted in place correspondingly, thus saving the subsequent step of manually assembling the first interface 1321 with the second interface 23; in addition, when the blood purification device 200 is in operation, the first interface 1321 can also be avoided from loosening due to movement relative to the monitoring panel 12, thereby improving the accuracy and reliability of the detection results of the blood purification device 200.

When the first interface 1321 is movably arranged relative to the monitoring panel 12, the movable arrangement allows a user to connect the first interface 1321 to the second interface 23 by operating the first interface 1321.

For example, after the monitoring panel 12 is mounted in place, the user can conveniently manually connect the first interface 1321 to the second interface 23, so as to avoid failure of alignment and sealing between the first interface 1321 and the second interface 23.

In one embodiment, in the actual production and manufacturing process, various parts inevitably have form tolerances, and in the assembly process of various parts, there are inevitably position tolerances between the various parts. In this regard, by arranging the first interface 1321 to be movable relative to the monitoring panel 12, after the monitoring panel 12 is mounted in place, the user only needs to manually operate to connect the first interface 1321 to the second interface 23, thus eliminating the need of a manufacturing process with high cost and high difficulty to ensure that the accumulated form and position tolerances of the first interface 1321, the monitoring panel 12, the blood purification device 200 and other parts are within an acceptable range, and ultimately reducing the manufacturing difficulty of the monitoring panel 12 and the difficulty of mounting the monitoring panel 12 on the device housing 21.

In addition, in the actual assembly process of mounting the panel kit 100 on the blood purification device 200, when there are too many parts such as the monitoring panel 12 and the first interface 1321 that need to be mounted in place at one time, it is difficult to ensure that all these parts can be mounted in place synchronously. Therefore, after the monitoring panel 12 is mounted in place, the user manually connects the first interface 1321 to the second interface 23, which can make the mounting of the panel kit 100 simpler and more convenient.

Optionally, when the first interface 1321 is movably arranged relative to the monitoring panel 12, during the process of mounting the monitoring panel 12 on the device housing 21, the first interface 1321 is movable relative to the monitoring panel 12, so as to compensate for the tolerance(s) of at least one of the first interface 1321, the monitoring panel 12, or the blood purification device 200.

Furthermore, by arranging the first interface 1321 to be movable relative to the monitoring panel 12, the failure to connect the second interface 23 to the first interface 1321 caused by the accumulated form and position tolerances of the first interface 1321, the monitoring panel 12, the blood purification device 200 and other parts during manufacturing and assembly can be avoided. At the same time, compared with the movable arrangement that allows the user to connect the first interface 1321 to the second interface 23 by operating the first interface 1321, in the embodiment of the present disclosure, by controlling the first interface 1321 to be movable only within a small range so as to compensate for the tolerance(s), the excessive movement of the first interface 1321 relative to the monitoring panel 12 can also be avoided, thereby avoiding the second interface 23 from colliding with the first interface 1321 and failing to be mounted in place. Of course, by avoiding the excessive movement of the first interface 1321 relative to the monitoring panel 12, shaking and loosening of the first interface 1321 during the operation of the blood purification device 200 can also be avoided, thereby improving the accuracy and reliability of the detection results of the blood purification device 200.

The above is an overall description of the technical solution of the embodiment of the present disclosure.

In some embodiments, the pump pipeline section 131 includes a first flexible pipeline, and when the pump pipeline section 131 is adapted to the pump assembly 22, the first flexible pipeline abuts against a pump head of the pump assembly 22, so that the rotation of the pump head can drive the first flexible pipeline to wind around the pump head. Therefore, during the mounting process of the panel kit 100, it is only necessary for the first flexible pipeline to contact the pump head of the pump assembly 22, and the user does not need to manually wind the pump pipeline section 131 around the pump head of the pump assembly 22, thus making the mounting of the pump pipeline section 131 simpler and more convenient.

For example, the pump assembly 22 may include a peristaltic pump, and when the first flexible pipeline contacts the pump head of the peristaltic pump, the peristaltic pump first drives the first flexible pipeline to wind around the pump head, and then the peristaltic pump continues to squeeze the first flexible pipeline through the rotation of the pump head, thereby driving the flow of the blood or the medical liquid in the pump pipeline section 131.

Optionally, the pump pipeline section 131 includes a first flexible pipeline, and when the pump pipeline section 131 is adapted to the pump assembly 22, the first flexible pipeline is wound around the pump head of the pump assembly 22, so that the rotation of the pump head directly drives the flow of the corresponding blood or medical liquid in the first flexible pipeline.

It can be understood that when the pump pipeline section 131 is adapted to the pump assembly 22, regardless of whether the first flexible pipeline is directly wound around the pump head of the pump assembly 22 or the pump assembly 22 needs to operate to wind the first flexible pipeline around the pump head of the pump assembly 22, it means that after the power panel 11 is mounted on the device housing 21, the user does not need to connect the pump pipeline section 131 to the pump assembly 22, thus making the mounting of the power panel 11 simpler.

The technical solution of the embodiment of the present disclosure is introduced below in combination with the mounting mode of the power panel 11.

In a first mounting mode of the power panel 11, when the power panel 11 is mounted at a first preset position of the device housing 21, the power panel 11 can be driven by the blood purification device 200 to move to a first target position, so that the pump pipeline section 131 is adapted to the pump assembly 22.

Furthermore, for the mounting of the panel kit 100, the user only needs to mount the power panel 11 at the first preset position of the device housing 21, and then the blood purification device 200 automatically drives the power panel 11 to the first target position to achieve the automatic mounting of the power panel 11, thus making the mounting of the panel kit 100 simpler.

Referring to Figs. 1 and 3, in some embodiments, the power panel 11 is provided with a first connecting part 111, and the first connecting part 111 is configured to be connectable to a first driving mechanism 24 on the device housing 21, so that the first driving mechanism 24 can drive the power panel 11 to move from a first preset position to a first target position.

The first connecting part 111 and the first driving mechanism 24 may be connected by means of clamping, screwing, magnetic fixing or the like, which is not limited in the embodiments of the present disclosure.

For example, if the first connecting part 111 can be fixed to the first driving mechanism 24 by clamping, the first driving mechanism 24 may be provided with a first claw 241, and the first claw 241 is fixed to the first connecting part 111 by clamping.

Correspondingly, the first connecting part 111 may include a first clamping hole, and the first claw 241 is clamped into the first clamping hole. Of course, the first claw 241 may alternatively include at least two first clamping arms, and the first connecting part 111 is a protruding structure on the power panel 11, so that the first connecting part 111 can be clamped between the two first clamping arms, which is not limited in the embodiments of the present disclosure.

In some embodiments, whether the power panel 11 is mounted at the first preset position and/or the first target position can be detected by a first position detection mechanism 25 on the blood purification device 200. Furthermore, the position of the power panel 11 can be accurately detected by the first position detection mechanism 25, so as to determine the overall position of the panel kit 100.

Thus, the blood purification device 200 can judge whether the power panel 11 is mounted in place when being mounted at the first preset position according to the detection result of the first position detection mechanism 25, so as to determine whether to drive the power panel 11 to move from the first preset position to the first target position through the first driving mechanism 24. In addition, after the blood purification device 200 drives the power panel 11 to move from the first preset position to the first target position through the first driving mechanism 24, the blood purification device 200 can also judge whether the power panel 11 is mounted in place according to the detection result of the first position detection mechanism 25, so as to avoid abnormal mounting of the power panel 11.

In some embodiments, when the power panel 11 is mounted at the first preset position and/or the first target position, the power panel 11 shields the first position detection mechanism 25 or contacts the first position detection mechanism 25, so that the first position detection mechanism 25 can detect the position of the power panel 11.

Exemplarily, the first position detection mechanism 25 may include a photoelectric sensor such as an infrared ranging sensor, a laser ranging sensor, a photoelectric gate and the like, so that the first position detection mechanism 25 can detect the position of the power panel 11 by detecting the distance to the power panel 11, whether the power panel 11 shields the corresponding photoelectric gate, and other ways.

Optionally, the first position detection mechanism 25 may also include a pressure sensor, so as to detect the position of the power panel 11 through the pressure exerted on the first position detection mechanism 25 when the power panel 11 is mounted at the first preset position and/or the first target position.

Of course, in some other embodiments, the first position detection mechanism 25 may further include a travel switch, a Hall sensor, a proximity switch and the like, which is not limited in the embodiments of the present disclosure.

It can also be understood that when the first position detection mechanism 25 is configured to detect whether the power panel 11 is mounted at the first preset position and the first target position, the first position detection mechanism 25 may include at least two first sensors, at least one first sensor is configured to detect whether the power panel 11 is mounted at the first preset position, and at least another first sensor is configured to detect whether the power panel 11 is mounted at the first target position.

The above is an introduction to the first mode of mounting the panel kit 100 in the embodiment of the present disclosure. The second mode of mounting the panel kit 100 in the embodiment of the present disclosure is described below.

In the second mode of mounting the power panel 11, the panel kit 100 can be manually mounted to mount the power panel 11 at the first target position and synchronously adapt the pump pipeline section 131 to the pump assembly 22. Furthermore, after the power panel 11 is mounted at the first target position, the user does not need to manually mount the pump pipeline section 131 at the pump head, thereby reducing the difficulty in mounting the panel kit 100 by the user.

Correspondingly, whether the power panel 11 is mounted at the first target position can be detected by the first position detection mechanism 25 on the blood purification device 200. Furthermore, the position of the power panel 11 can be accurately detected by the first position detection mechanism 25, so as to determine the overall position of the panel kit 100.

The specific structure of the first position detection mechanism 25 will not be repeated here.

In some embodiments, the power panel 11 may be provided with a first connecting part 111, and the first connecting part 111 is configured to be connectable to the device housing 21, so as to achieve the detachable mounting of the power panel 11 at the first target position.

For example, a first claw 241 may be fixedly arranged on the device housing 21, and the specific structures of the first claw 241 and the first connecting part 111 will not be repeated here.

The above is a brief introduction to the second mode of mounting the panel kit 100 in the embodiment of the present disclosure, and the technical solution of the embodiment of the present disclosure is further described below in combination with the monitoring panel 12 in the embodiment of the present disclosure.

In the first mode of mounting the monitoring panel 12, the panel kit 100 can be manually mounted to mount the monitoring panel 12 at a second target position of the device housing 21 and synchronously connect the first interface 1321 to the second interface 23. If the first interface 1321 is connected to the second interface 23, the monitoring pipeline section 132 is used for the blood purification device 200 to monitor parameter information. This can reduce the step of separately mounting the first interface 1321 and avoid the user from mounting the first interface 1321 at a wrong position when manually mounting it, thereby improving the mounting accuracy of the first interface 1321.

It should be noted that the panel kit 100 can be manually mounted to mount the monitoring panel 12 at the second target position of the device housing 21 and synchronously connect the first interface 1321 to the second interface 23, which can also be understood as that the user can make the monitoring panel 12 drive the connection of the first interface 1321 to the second interface 23 synchronously through the action of mounting the monitoring panel 12 at the second target position. In this process, the two actions of mounting the monitoring panel 12 at the second target position and connecting the first interface 1321 to the second interface 23 can be completed simultaneously or with a certain time difference. For example, when the first interface 1321 is movably arranged relative to the monitoring panel 12, after the first interface 1321 is connected to the second interface 23, the monitoring panel 12 may continue to move a certain distance to be mounted at the second target position of the device housing 21, which is not limited in the embodiments of the present disclosure.

In the second mode of mounting the monitoring panel 12, if the monitoring panel 12 is mounted at the second target position of the device housing 21, the second interface 23 can be extended to be connected to the first interface 1321, and if the first interface 1321 is connected to the second interface 23, the monitoring pipeline section 132 is used for the device housing 21 to monitor parameter information. Thus, the user only needs to simply mount the monitoring panel 12 at the second target position of the device housing 21, and the second interface 23 on the blood purification device 200 can be extended to be connected to the first interface 1321 of the monitoring pipeline section 132, and the user does not need to manually connect the second interface 23 to the first interface 1321 after mounting the monitoring panel 12 at the second target position of the device housing 21, making the mounting of the panel kit 100 simpler.

In some embodiments, when the monitoring panel 12 is mounted at a second preset position of the device housing 21, the device housing 21 can drive the monitoring panel 12 to move to the second target position.

Optionally, the user may also directly mount the monitoring panel 12 at the second target position of the device housing 21, which is not limited in the embodiments of the present disclosure.

In the third mode of mounting the monitoring panel 12, if the monitoring panel 12 is mounted at the second preset position of the device housing 21, the blood purification device 200 can drive the monitoring panel 12 to move to the second target position, so that the first interface 1321 is fixedly connected to the second interface 23. When the first interface 1321 is connected to the second interface 23, the monitoring pipeline section 132 is used for the blood purification device 200 to monitor parameter information. Furthermore, for the mounting of the panel kit 100, the user only needs to mount the monitoring panel 12 at the second preset position of the device housing 21, and then the blood purification device 200 automatically drives the monitoring panel 12 to the first target position, so as to achieve the automatic mounting of the monitoring panel 12, thus making the mounting of the panel kit 100 simpler.

In the fourth mode of mounting the monitoring panel 12, the first interface 1321 is movably arranged relative to the monitoring panel 12. If the monitoring panel 12 is mounted at the second target position of the device housing 21, the first interface 1321 can be manually operated to achieve the connection between the first interface 1321 and the second interface 23. When the second interface 23 is connected to the first interface 1321, the monitoring pipeline section 132 is used for the blood purification device 200 to monitor parameter information.

After the monitoring panel 12 is mounted in place, the user can conveniently manually connect the first interface 1321 to the second interface 23 to avoid failure of alignment and sealing between the first interface 1321 and the second interface 23.

In one embodiment, in the actual production and manufacturing process, various parts inevitably have form tolerances, and in the assembly process of various parts, there are inevitably position tolerances between the various parts. In this regard, after the monitoring panel 12 is mounted at the second target position, the user only needs to manually operate to connect the first interface 1321 to the second interface 23, thus eliminating the need of a manufacturing process with high cost and high difficulty to ensure that the accumulated form and position tolerances of the first interface 1321, the monitoring panel 12, the blood purification device 200 and other parts are within an acceptable range, so as to achieve the one-time mounting of the first interface 1321 and the monitoring panel 12 in place, and ultimately reducing the manufacturing difficulty of the panel kit 100 and the difficulty of mounting the panel kit 100 on the device housing 21.

In addition, in the actual assembly process of mounting the panel kit 100 on the blood purification device 200, when there are too many parts such as the monitoring panel 12 and the first interface 1321 that need to be mounted in place at one time, it is difficult to ensure that all these parts can be mounted in place synchronously. Therefore, after the monitoring panel 12 is mounted in place, the user manually connects the first interface 1321 to the second interface 23, which can make the mounting of the panel kit 100 simpler and more convenient.

In one embodiment, the connection between the first interface 1321 and the second interface 23 may be a sealed connection.

It can also be understood that in the fourth mode of mounting the monitoring panel 12, the user may manually mount the monitoring panel 12 at the second preset position, and then the blood purification device 200 automatically drives the monitoring panel 12 to the second target position to complete the mounting, or the user may directly mount the monitoring panel 12 at the second target position to complete the mounting, which is not limited in the embodiments of the present disclosure.

In some embodiments, the first interface 1321 and the second interface 23 may be connected by means of screwing, clamping, plugging and the like. Based on this, when the monitoring panel 12 is mounted at the second target position, the first interface 1321 can be manually operated by tightening the first interface 1321, pressing the first interface 1321, plugging the first interface 1321 and other ways to achieve the connection between the first interface 1321 and the second interface 23, which is not limited in the embodiments of the present disclosure.

For example, the first interface 1321 is provided with a knob. If the monitoring panel 12 is mounted at the second target position, the first interface 1321 can be connected to the second interface 23 by the user turning the knob.

The above is an overall introduction to the four modes of mounting the monitoring panel 12 in the embodiment of the present disclosure.

In some embodiments, if the monitoring panel 12 is at the second preset position, the first interface 1321 is not connected to the second interface 23 on the device housing 21. Furthermore, if the monitoring panel 12 is at the second preset position, a certain gap may be reserved between the first interface 1321 and the second interface 23, so that when the monitoring panel 12 moves toward the second target position, the first interface 1321 can move toward the second interface 23 correspondingly.

In addition, when the user needs to replace the panel kit 100 on the device housing 21 (i.e., needs to remove the panel kit 100 from the device housing 21), the device housing 21 can drive the power panel 11 to move from the second target position to the second preset position, so that the first interface 1321 is separated from the second interface 23, making it more convenient for the user to detach the panel kit 100 from the device housing 21.

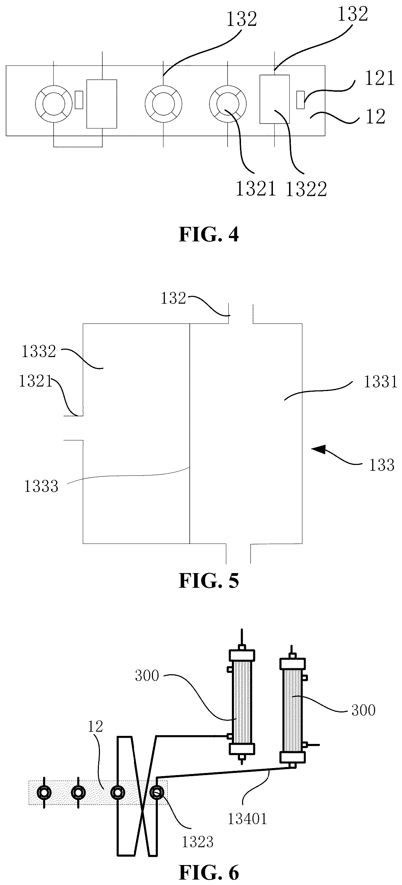

Referring to Figs. 1 and 4, in some embodiments, whether the monitoring panel 12 is mounted at the second preset position and/or the second target position can be detected by a second position detection mechanism 26 on the blood purification device 200.

Thus, the blood purification device 200 can judge whether the monitoring panel 12 is mounted in place when being mounted at the second preset position according to the detection result of the second position detection mechanism 26, so as to determine whether to drive the monitoring panel 12 to move from the second preset position to the second target position; in addition, after the blood purification device 200 drives the power panel 11 to move from the second preset position to the second target position, the blood purification device 200 can also judge whether the monitoring panel 12 is mounted in place according to the detection result of the second position detection mechanism 26.

In some embodiments, when the monitoring panel 12 is mounted at the second preset position and/or the second target position, the monitoring panel 12 shields the second position detection mechanism 26 or contacts the second position detection mechanism 26, so that the second position detection mechanism 26 can detect the position of the monitoring panel 12.

Exemplarily, the second position detection mechanism 26 may include a photoelectric sensor such as an infrared ranging sensor, a laser ranging sensor, a photoelectric gate and the like, so that the second position detection mechanism 26 can detect the position of the monitoring panel 12 by detecting the distance to the monitoring panel 12, whether the monitoring panel 12 shields the corresponding photoelectric gate, and other methods.

Optionally, the second position detection mechanism 26 may further include a pressure sensor, so as to detect the position of the monitoring panel 12 through the pressure exerted on the second position detection mechanism 26 when the monitoring panel 12 is mounted at the second target position.

Of course, in some other embodiments, the second position detection mechanism 26 may further include a travel switch, a Hall sensor, a proximity switch and the like, which is not limited in the embodiments of the present disclosure.

It can also be understood that in the first mode of mounting the monitoring panel 12, the second position detection mechanism 26 on the blood purification device 200 may only be configured to detect whether the monitoring panel 12 is at the second target position, that is, whether the monitoring panel 12 is mounted at the second target position can be detected by the second position detection mechanism 26 on the blood purification device 200.

It can be understood that when the second position detection mechanism 26 is configured to detect whether the monitoring panel 12 is mounted at the second preset position and the second target position, the second position detection mechanism 26 may include at least two second sensors, at least one of the second sensors is configured to detect whether the monitoring panel 12 is mounted at the second preset position, and at least another one of the second sensors is configured to detect whether the monitoring panel 12 is mounted at the second target position.

In some embodiments, the monitoring panel 12 is provided with a second connecting part 121, and the second connecting part 121 is configured to be connectable to a second driving mechanism 27 of the blood purification device 200, so that the second driving mechanism 27 can drive the monitoring panel 12 to move from the second preset position to the second target position.

For example, the second driving mechanism 27 is provided with a second claw 271, and the second driving mechanism 27 can drive the second claw 271 to move. The second claw 271 is fixed to the second connecting part 121 by clamping.

Exemplarily, the second connecting part 121 may include a second clamping hole, and the second claw 271 can be clamped into the second clamping hole. Of course, the second claw 271 may include at least two second clamping arms, and the second connecting part 121 is a protruding structure on the monitoring panel 12, so that the second connecting part 121 can be clamped between the two second clamping arms, which is not limited in the embodiments of the present disclosure.

It can also be understood that the second claw 271 may be fixedly arranged on the device housing 21, and correspondingly, the blood purification device 200 is not provided with the second driving mechanism 27, so that the user can directly mount the monitoring panel 12 at the second target position, which is not limited in the embodiments of the present disclosure.

In the below, the technical solution of the embodiments of the present disclosure is further described in conjunction with some other structures of the monitoring panel 12.

In some embodiments, the first interface 1321 is fixedly (i.e., non-detachably) connected to the corresponding monitoring pipeline section 132. For example, the first interface 1321 and the corresponding monitoring pipeline section 132 may be connected by bonding, integral molding, fusion, welding or other means, so as to avoid inaccurate detection results of the blood purification device 200 caused by loosening or even falling off of the first interface 1321.

Optionally, in some other embodiments, the first interface 1321 is detachably connected to the corresponding monitoring pipeline section 132. For example, the first interface 1321 and the corresponding monitoring pipeline section 132 are connected by screwing, clamping, magnetic fixing or other means, so as to facilitate replacement of a suitable first interface 1321 according to the model change of the second interface 23 of the blood purification device 200.

In some embodiments, the first interface 1321 is connected to the corresponding monitoring pipeline section 132 through a connector. The connector may be a Luer connector or other types of connectors, which is not limited in the embodiments of the present disclosure.

In some embodiments, the monitoring pipeline section 132 is fixedly (i.e., non-detachably) connected to the monitoring panel 12. For example, the monitoring pipeline section 132 and the monitoring panel 12 may be connected by bonding, integral molding, fusion, welding or other means, so as to avoid inaccurate detection results of the blood purification device 200 caused by loosening or even falling off of the monitoring pipeline section 132.

Optionally, the monitoring pipeline section 132 may also be detachably connected to the monitoring panel 12. For example, the monitoring pipeline section 132 and the monitoring panel 12 are connected by screwing, clamping, magnetic fixing or other means, so as to facilitate replacement of a suitable monitoring pipeline section 132 or monitoring panel 12 according to the change of the blood purification device 200.

In some embodiments, the flow path connected between the pump pipeline section 131 and the monitoring pipeline section 132 is a flexible pipe.

For example, the pipeline assembly 13 further includes a second flexible pipeline, and the second flexible pipeline is connected between at least one pump pipeline section 131 and at least one monitoring pipeline section 132.

It can be understood that when the pump assembly 22 of the device housing 21 drives the pump pipeline section 131 to operate, the pump assembly 22 will not only cause the vibration of the power panel 11 but also pull the pump pipeline section 131. Therefore, on the basis that the power panel 11 and the monitoring panel 12 are arranged separately to prevent the vibration of the power panel 11 from being transferred to the monitoring panel 12, the second flexible pipeline can be adaptively deformed when the pump pipeline section 131 is pulled, so as to avoid the pump pipeline section 131 from pulling the connected monitoring pipeline section 132, thereby avoiding the loosening of the first interface 1321 due to the interference of the connected second flexible pipeline and/or the monitoring panel 12, and ultimately improving the accuracy of the detection results of the blood purification device 200.

With continued reference to Fig. 5, in some embodiments, at least one monitoring pipeline section 132 includes a pressure monitoring module 133, and the pressure monitoring module 133 includes a fluid part 1331, a gas part 1332, a diaphragm 1333 and a first interface 1321. A fluid-side cavity defined at least in part by the fluid part 1331 and a gas-side cavity defined at least in part by the gas part 1332 are separated by the diaphragm 1333, the fluid-side cavity is used for the flow of the blood or the medical liquid, the first interface 1321 is connected to the gas-side cavity, and if the first interface 1321 is connected to the second interface 23, the monitoring pipeline section 132 is used for the device housing 21 to monitor the pressure parameter of the blood or medical liquid flowing through the fluid-side cavity.

Thus, when blood or medical liquid flows through the fluid-side cavity, the blood or medical liquid will squeeze the diaphragm 1333, resulting in a change in air pressure in the gas-side cavity. Furthermore, the second interface 23 on the device housing 21 is connected to the first interface 1321 and thus can monitor the air pressure in the gas-side cavity, so as to monitor the pressure parameter of the corresponding blood or medical liquid, and thus the second interface 23 does not need to contact the corresponding blood or medical liquid, thereby avoiding contamination of the blood or medical liquid.

Of course, in some other embodiments, at least one monitoring pipeline section 132 may be adapted to a contact pressure sensor on the blood purification device 200. Thus, the contact pressure sensor on the device housing 21 can directly abut against the diaphragm 1333 to monitor the pressure parameter of the corresponding blood or medical liquid, which is not limited in the embodiments of the present disclosure.

In some embodiments, at least two monitoring pipeline sections 132 are provided with the first interface 1321, and if the first interface 1321 is connected to the second interface 23, the monitoring pipeline section 132 is used for the blood purification device 200 to monitor pressure parameter. Thus, the connection of at least two first interfaces 1321 for monitoring pressure parameter can be achieved through the monitoring panel 12, making the mounting of the panel kit 100 more convenient.

In some embodiments, the filter 300 is configured to filter the blood flowing through the pump pipeline section 131.

Here, the filter 300 may be arranged on the blood purification device 200, and the panel kit 100 is connected to the filter 300 on the blood purification device 200 after the panel kit 100 is mounted on the device housing 21. Alternatively, the filter 300 may be arranged on the panel kit 100, and the filter 300 is fixed to the device housing 21 during the process of mounting the panel kit 100 on the device housing 21, which is not limited in the embodiments of the present disclosure.

As shown in Fig. 4, in some embodiments, if the first interface 1321 is connected to the second interface 23, the monitoring pipeline section 132 is used for the blood purification device 200 to monitor the pressure parameter of the corresponding blood or medical liquid. The at least two monitoring pipeline sections 132 further include a blood leakage monitoring pipeline section 1322, which is configured to cooperate with a blood leakage monitoring module on the blood purification device 200 to monitor whether the blood on one side of the filter membrane of the filter 300 permeates to the other side. Thus, the connection of at least one first interface 1321 for monitoring pressure parameter and the mounting of the blood leakage monitoring pipeline section 1322 can be achieved through the monitoring panel 12, making the mounting of the panel kit 100 more convenient.

With continued reference to Fig. 6, in some embodiments, the monitoring pipeline section 132 further includes a secondary membrane pressure monitoring pipeline section 1323, which is configured to connect the blood output of one filter 300 to the blood input of the other filter 300, and the secondary membrane pressure monitoring pipeline section 1323 is configured to monitor the pressure parameter of the blood.

For example, the pipeline assembly 13 may further include a connecting flow path 13401, which is connected to the blood output of one filter 300 and the blood input of the other filter 300 respectively, and the secondary membrane pressure monitoring pipeline section 1323 is arranged on the connecting flow path 13401.

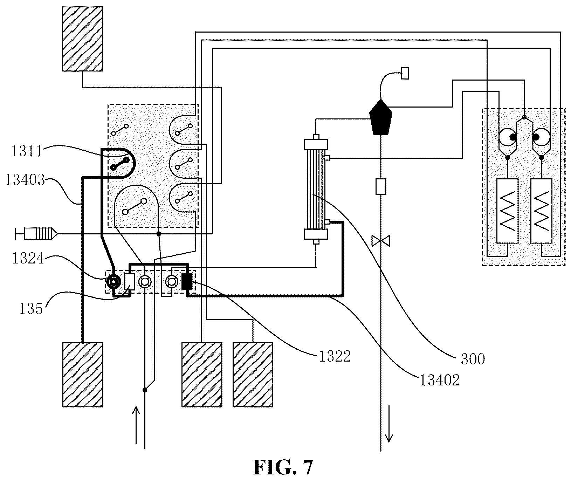

With continued reference to Fig. 7, in some embodiments, the pump pipeline section 131 includes a waste liquid pressure pump pipeline section 1311. The pipeline assembly 13 further includes a waste liquid flow path 13402, the output of the waste liquid flow path 13402 is connected to the input of the waste liquid pressure pump pipeline section 1311, and the input of the waste liquid flow path 13402 is configured to be connected to the medical liquid output of the filter 300. The monitoring pipeline section 132 further includes a waste liquid pressure monitoring pipeline section 1324, which is arranged on the waste liquid flow path 13402 and configured to monitor the pressure parameter of the medical liquid.

Therefore, when the pump assembly 22 is in operation, the medical liquid in the filter 300 can flow along the waste liquid flow path 13402 to the waste liquid pressure pump pipeline section 1311, passing through the waste liquid pressure monitoring pipeline section 1324. Thus, the waste liquid pressure monitoring pipeline section 1324 is configured to monitor the pressure parameter of the medical liquid discharged from the filter 300 (hereinafter referred to as waste liquid pressure for short). Furthermore, it is possible to judge whether the filter membrane of the filter 300 is clogged by monitoring the waste liquid pressure.

It should be noted here that, compared with arranging the waste liquid pressure monitoring pipeline section 1324 downstream of the waste liquid pressure pump pipeline section 1311, in the embodiments of the present disclosure, the waste liquid pressure monitoring pipeline section 1324 is arranged between the waste liquid pressure pump pipeline section 1311 and the filter 300, which can prevent other failures downstream of the waste liquid pressure pump pipeline section 1311 from interfering with the monitoring of whether the filter membrane of the filter 300 is clogged by the waste liquid pressure monitoring pipeline section 1324.

In some embodiments, the pipeline assembly 13 further includes a collection flow path 13403, the input of the collection flow path 13403 is connected to the output of the waste liquid pressure pump pipeline section 1311, and the output of the collection flow path 13403 is configured to be connected to a medical liquid collection container or the secondary membrane pressure monitoring pipeline section 1323. Thus, the medical liquid in the filter 300 can flow into the medical liquid collection container or the secondary membrane pressure monitoring pipeline section 1323 after flowing through the waste liquid flow path 13402, the waste liquid pressure pump pipeline section 1311 and the collection flow path 13403 in sequence.

In some embodiments, the monitoring pipeline section 132 further includes a blood leakage monitoring pipeline section 1322, which is arranged on the waste liquid flow path 13402 or the collection flow path 13403. If the blood leakage monitoring pipeline section 1322 is adapted to the blood leakage monitoring module on the device housing 21, the blood leakage monitoring pipeline section 1322 is used for the blood leakage monitoring module to monitor whether the blood on one side of the filter membrane of the filter 300 permeates to the other side.

Exemplarily, the blood leakage monitoring module on the device housing 21 may include a photoelectric sensor such as a color sensor, that is configured to judge the presence of the blood compositions by monitoring the color of the medical liquid discharged from the filter 300.

Correspondingly, at least a partial region of the blood leakage monitoring pipeline section 1322 may be made of a light-shielding material to avoid ambient light from affecting the detection result of the blood leakage monitoring module.

Of course, the blood leakage monitoring module may also be any other module for detecting blood compositions, which is not limited in the embodiments of the present disclosure.

It can also be understood that the input of the blood leakage monitoring pipeline section 1322 may face downward, so that the medical liquid flows through the blood leakage monitoring pipeline section 1322 from bottom to top, thereby avoiding the accumulation of air bubbles when the medical fluid flows through the blood leakage monitoring section 1322 from top to bottom, which may affect the detection result of the blood leakage monitoring module. Of course, in some other embodiments, the input of the blood leakage monitoring pipeline section 1322 may also face other directions, which is not limited in the embodiments of the present disclosure.

Similarly, the input of at least one monitoring pipeline section 132 may face downward, so that the corresponding medical liquid or blood flows through the monitoring pipeline section 132 from bottom to top, thereby avoiding the accumulation of air bubbles in the monitoring pipeline section 132, which may affect the detection result.

Optionally, in some other embodiments, the panel kit 100 includes a waste liquid branch, which is configured to be connected to the medical liquid output of the filter 300. The waste liquid branch is provided with a blood leakage monitoring pipeline section 1322, and the waste liquid branch allows the medical liquid flowing out of the filter 300 to pass through the blood leakage monitoring pipeline section 1322. The blood purification device 200 further includes a blood leakage monitoring module, which can monitor whether the blood on one side of the filter membrane of the filter 300 permeates to the other side through the blood leakage monitoring pipeline section 1322.

For example, the waste liquid branch includes a waste liquid flow path 13402 and a collection flow path 13403. Then, the blood leakage monitoring pipeline section 1322 may be arranged on the waste liquid flow path 13402 or the collection flow path 13403, which is not limited in the embodiments of the present disclosure.

In some embodiments, the waste liquid branch allows the medical liquid flowing out of the filter 300 to flow through the blood leakage monitoring pipeline section 1322 from bottom to top, thereby avoiding the accumulation of air bubbles in the blood leakage monitoring pipeline section 1322, which may affect the detection result.

It can also be understood that the blood leakage monitoring pipeline section 1322 may be arranged on the monitoring panel 12, thus serving as one of the monitoring pipeline sections 132; alternatively, the blood leakage monitoring pipeline section 1322 may not be arranged on the monitoring panel 12, for example, the blood leakage monitoring pipeline section 1322 may be arranged between the power panel 11 and the monitoring panel 12, which is not limited in the embodiments of the present disclosure.

Exemplarily, the blood leakage monitoring pipeline section 1322 may be arranged on the waste liquid flow path 13402, between the input of the waste liquid pressure pump pipeline section 1311 and the output of the waste liquid pressure monitoring pipeline section 1324.

Thus, on the one hand, it is possible to avoid the need of reserving a large space on the monitoring panel 12 for mounting the blood leakage monitoring pipeline section 1322, which is conducive to the miniaturization design of the monitoring panel 12; on the other hand, the number of monitoring pipeline sections 132 on the monitoring panel 12 can be reduced, so that the pipelines of the panel kit 100 do not need to be bent multiple times on the monitoring panel 12 to form a large number of monitoring pipeline sections 132, thereby not only reducing the manufacturing difficulty of the panel kit 100 but also making the flow of the corresponding blood or medical liquid in the panel kit 100 smoother.

In addition, when the input of the waste liquid pressure monitoring pipeline section 1324 also faces downward, the input of the blood leakage monitoring pipeline section 1322 may also face downward to connect to the upwardly arranged output of the waste liquid pressure monitoring pipeline section 1324, thereby avoiding the accumulation of air bubbles when the medical liquid flows through the blood leakage monitoring pipeline section 1322 from top to bottom, which may affect the detection result of the blood leakage monitoring module.

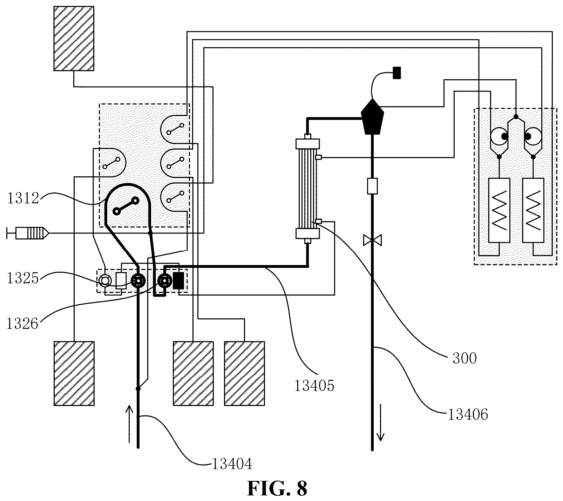

With continued reference to Fig. 8, in some embodiments, the pump pipeline section 131 includes a blood pump pipeline section 1312. The pipeline assembly 13 further includes a blood drawing flow path 13404, a blood pumping flow path 13405 and a blood return flow path 13406. The input of the blood drawing flow path 13404 is configured to be connected to a blood drawing site of a blood vessel of a patient, and the output of the blood drawing flow path 13404 is connected to the input of the blood pump pipeline section 1312. The input of the blood pumping flow path 13405 is connected to the output of the blood pump pipeline section 1312, and the output of the blood pumping flow path 13405 is configured to connect to the blood input of the filter 300. The input of the blood return flow path 13406 is configured to be connected to the blood output of the filter 300, and the output of the blood return flow path 13406 is configured to be connected to a blood return site of the blood vessel of the patient.

Thus, during the operation of the blood purification device 200, when the pump assembly 22 of the device housing 21 is in operation, the blood pump pipeline section 1312 drives a blood to flow through the blood drawing flow path 13404, the blood pump pipeline section 1312, the blood pumping flow path 13405, the filter 300 and the blood return flow path 13406 in sequence from the blood drawing site of the blood vessel of the patient, and then flow back to the blood vessel of the patient from the blood return site of the patient's blood vessel, thereby realizing extracorporeal blood circulation therapy for the patient.

In some embodiments, the monitoring pipeline section 132 includes a blood drawing pressure monitoring pipeline section 1325, that is arranged on the blood drawing flow path 13404 and configured to monitor the pressure parameter of the blood.

Thus, the blood drawing pressure monitoring pipeline section 1325 can be configured to monitor the pressure parameter of blood drawn from the blood vessel of the patient (hereinafter referred to as blood drawing pressure for short). Specifically, when the pump assembly 22 is in operation, the blood pump pipeline section 1312 can drive the blood to flow from the blood vessel of the patient into the blood drawing flow path 13404, and then the blood flows through the blood drawing pressure monitoring pipeline section 1325 when moving along the blood drawing flow path 13404, so that the blood drawing pressure monitoring pipeline section 1325 detects the blood drawing pressure. Then, the blood purification device 200 can judge whether blood is normally drawn from the blood vessel of the patient by monitoring the blood drawing pressure.

In some embodiments, the monitoring pipeline section 132 further includes a pressure monitoring pipeline section before filtering 1326, that is arranged on the blood pumping flow path 13405 and configured to monitor the pressure parameter of blood.

Thus, the pressure monitoring pipeline section before filtering 1326 can be configured to monitor the pressure parameter of blood flowing into the filter 300 from the pump pipeline section 131 (hereinafter referred to as pressure before filtering for short). Furthermore, it is possible to judge whether blood can normally flow into the filter 300 by monitoring the pressure before filtering. For example, if the pressure before filtering is excessively high, it may indicate that clogging, blood coagulation or other conditions have occurred in the filter 300.

In some embodiments, the number of the monitoring panels 12 is at least two, and the monitoring panels 12 are arranged separately from each other, so that each monitoring panel 12 can be detached and mounted separately, and each monitoring panel 12 is provided with at least two different types of pipeline sections among the blood leakage monitoring pipeline section 1322, the blood drawing pressure monitoring pipeline section 1325, the pressure monitoring pipeline section before filtering 1326 and the waste liquid pressure monitoring pipeline section 1324. Thus, with at least two monitoring panels 12, the panel kit 100 can be adapted to the mounting requirements of more different types of device housings 21.

Exemplarily, one monitoring panel 12 may be provided with the blood drawing pressure monitoring pipeline section 1325 and the pressure monitoring pipeline section before filtering 1326, and another monitoring panel 12 may be provided with the blood leakage monitoring pipeline section 1322 and the waste liquid pressure monitoring pipeline section 1324, which is not limited in the embodiments of the present disclosure.

When the monitoring pipeline section 132 further includes the secondary membrane pressure monitoring pipeline section 1323, the number of the monitoring panels 12 may also be at least two, and the monitoring panels 12 are arranged separately from each other, so that each monitoring panel 12 can be detached and mounted separately, and each monitoring panel 12 is provided with at least two different types of pipeline sections among the blood leakage monitoring pipeline section 1322, the blood drawing pressure monitoring pipeline section 1325, the pressure monitoring pipeline section before filtering 1326, the waste liquid pressure monitoring pipeline section 1324 and the secondary membrane pressure monitoring pipeline section 1323. Thus, with at least two monitoring panels 12, the panel kit 100 can be adapted to the mounting requirements of more different types of device housings 21.

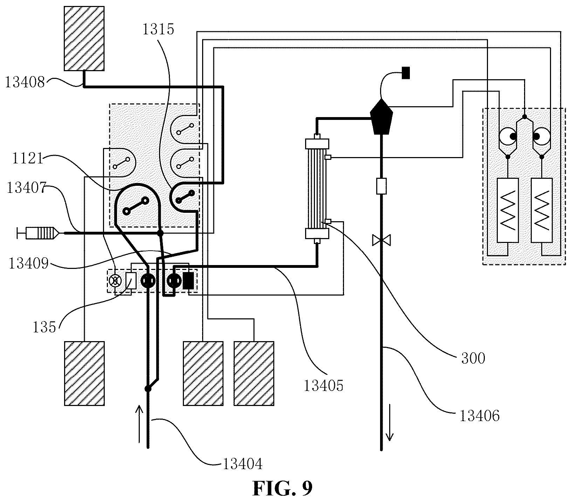

Please continue to refer to Fig. 9, in some embodiments, the pipeline assembly 13 further includes an injection flow path 13407. The input of the injection flow path 13407 is configured to be connected to an anticoagulant injection pump, and the output of the injection flow path 13407 is connected to the blood drawing flow path 13404 or the blood pumping flow path 13405. Thus, the anticoagulant injection pump provided on the blood purification device 200 can inject an anticoagulant into the blood in the blood drawing flow path 13404 or the blood pumping flow path 13405 through the injection flow path 13407, so as to prevent blood coagulation in the blood drawing flow path 13404 or the blood pumping flow path 13405.

The anticoagulant may include heparin, citric acid, etc., which is not limited in the embodiments of the present disclosure.

It can also be understood that when the injection flow path 13407 is connected to the blood pumping flow path 13405, to a certain extent, it can prevent a large negative pressure from being generated in the blood drawing flow path 13404 when the pump assembly 22 is in operation, which would cause the anticoagulant in the anticoagulant injection pump to be aspirated out.

In some embodiments, the pump pipeline section 131 further includes a front pump pipeline section 1315 before blood pump (referred to front pump pipeline section for short hereinafter). The pipeline assembly 13 includes a first liquid inlet flow path 13408 and a second liquid inlet flow path 13409. The first liquid inlet flow path 13408, the front pump pipeline section 1315, and the second liquid inlet flow path 13409 are connected in sequence. The input of the first liquid inlet flow path 13408 is at least configured to be connected to an anticoagulant supply container, and the output of the second liquid inlet flow path 13409 is connected to the blood drawing flow path 13404, the blood pumping flow path 13405 or the blood return flow path 13406. Thus, the device housing 21 can drive the front pump pipeline section 1315 through the pump assembly 22, so that the anticoagulant in the anticoagulant supply container flows into the blood drawing flow path 13404, the blood pumping flow path 13405 or the blood return flow path 13406 through the first liquid inlet flow path 13408, the front pump pipeline section 1315 and the second liquid inlet flow path 13409 in sequence, thereby preventing blood coagulation in the blood drawing flow path 13404, the blood pumping flow path 13405 or the blood return flow path 13406.

It can be understood that when the pipeline assembly 13 is provided with the injection flow path 13407, the first liquid inlet flow path 13408, the front pump pipeline section 1315 and the second liquid inlet flow path 13409 at the same time, the anticoagulant injection pump connected to the injection flow path 13407 and the anticoagulant supply container connected to the first liquid inlet flow path 13408 may be configured to supply different anticoagulants. For example, the anticoagulant injection pump is configured to supply heparin, and the anticoagulant supply container is configured to supply citric acid, so as to meet different anticoagulation requirements in the extracorporeal blood purification process.

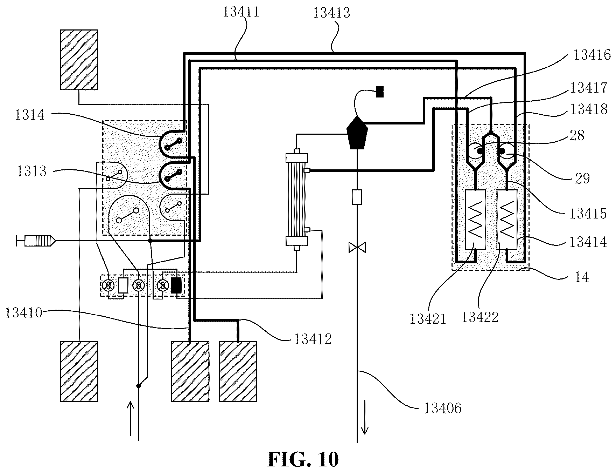

Please continue to refer to Fig. 10, in some embodiments, the pump pipeline section 131 includes a dialysate pump pipeline section 1313. The pipeline assembly 13 further includes a third liquid inlet flow path 13410 and a fourth liquid inlet flow path 13411. The third liquid inlet flow path 13410, the dialysate pump pipeline section 1313, and the fourth liquid inlet flow path 13411 are connected in sequence. The input of the third liquid inlet flow path 13410 is configured to be connected to a dialysate supply container, and the output of the fourth liquid inlet flow path 13411 is configured to be connected to the medical liquid input of the filter 300.

Thus, when the pump assembly 22 on the device housing 21 is in operation, the pump assembly 22 can drive, through the dialysate pump pipeline section 1313, the dialysate in the dialysate supply container to flow through the third liquid inlet flow path 13410, the dialysate pump pipeline section 1313, the fourth liquid inlet flow path 13411 and the medical liquid input of the filter 300 in sequence, and then flow into the filter 300.

In some embodiments, the pump pipeline section 131 further includes a replacement fluid pump pipeline section 1314. The pipeline assembly 13 further includes a fifth liquid inlet flow path 13412 and a sixth liquid inlet flow path 13413. The fifth liquid inlet flow path 13412, the replacement fluid pump pipeline section 1314, and the sixth liquid inlet flow path 13413 are connected in sequence. The input of the fifth liquid inlet flow path 13412 is configured to be connected to a replacement fluid supply container, and the output of the sixth liquid inlet flow path 13413 is connected to the blood drawing flow path 13404, the blood pumping flow path 13405 or the blood return flow path 13406.

Thus, when the pump assembly 22 on the device housing 21 is in operation, the pump assembly 22 can drive, through the replacement fluid pump pipeline section 1314, the replacement fluid in the replacement fluid supply container to flow through the fifth liquid inlet flow path 13412, the replacement fluid pump pipeline section 1314 and the sixth liquid inlet flow path 13413 in sequence, and then flow into the blood drawing flow path 13404, the blood pumping flow path 13405 or the blood return flow path 13406.

The replacement fluid may include commercial replacement fluids, such as online replacement fluid produced by a hemodiafiltration machine and manually configured replacement fluid, etc., which is not limited in the embodiments of the present disclosure.

In some embodiments, the panel kit 100 further includes a heating panel 14, which is configured to be detachably mounted to the heating region of the device housing 21. The pipeline assembly 13 includes a heating container 13414 and a liquid outlet flow path 13415, the liquid outlet flow path 13415 is connected to the output of the heating container 13414, and the heating container 13414 is mounted on the heating panel 14 so as to be mounted to the heating region through the heating panel 14, for the heating device of the blood purification device 200 to heat the blood and/or medical liquid in the pipeline assembly 13. Thus, the heating requirements of blood and/or medical liquid can be met. For example, the medical liquid can be heated to avoid the impact of low-temperature medical liquid on blood, such as discomfort caused by low-temperature blood flowing back into the human body due to low-temperature medical liquid.

It can be understood that the heating container 13414 may include a coil-type heating container, a capsule-type heating container, a bottle-type heating container, and a bag-type heating container, etc., which is not limited in the embodiments of the present disclosure.

In some embodiments, when the pump pipeline section 131 includes the dialysate pump pipeline section 1313, the heating container 13414 includes a first heating container 13421. The input of the first heating container 13421 is connected to the output of the fourth liquid inlet flow path 13411, and the liquid outlet flow path 13415 connected to the first heating container 13421 includes a first liquid outlet flow path 13416 and a second liquid outlet flow path 13417. The first liquid outlet flow path 13416 is connected to the blood return flow path 13406, and the second liquid outlet flow path 13417 is configured to be connected to the medical liquid input of the filter 300.

Thus, in actual use, the input of the third liquid inlet flow path 13410 can be selectively connected to a dialysate supply container or a replacement fluid supply container, so that the blood purification device 200 can meet more different usage requirements.

For example, when the input of the third liquid inlet flow path 13410 is connected to a dialysate supply container, the first pipeline switching device 28 of the blood purification device 200 can be used to control the first liquid outlet flow path 13416 to be disconnected and the second liquid outlet flow path 13417 to be connected, so that the dialysate in the dialysate supply container flows through the third liquid inlet flow path 13410, the dialysate pump pipeline section 1313, the fourth liquid inlet flow path 13411, the first heating container 13421, the second liquid outlet flow path 13417 and the medical liquid input of the filter 300 in sequence, and then flows into the filter 300.

Alternatively, when the input of the third liquid inlet flow path 13410 is connected to a replacement fluid supply container, the first pipeline switching device 28 of the blood purification device 200 can be used to control the first liquid outlet flow path 13416 to be connected and the second liquid outlet flow path 13417 to be disconnected, so that the replacement fluid in the replacement fluid supply container flows through the third liquid inlet flow path 13410, the dialysate pump pipeline section 1313, the fourth liquid inlet flow path 13411, the first heating container 13421 and the first liquid outlet flow path 13416 in sequence, and then flows into the blood return flow path 13406.

In some embodiments, when the pump pipeline section 131 includes the replacement fluid pump pipeline section 1314, the heating container 13414 includes a second heating container 13422. The input of the second heating container 13422 is connected to the output of the sixth liquid inlet flow path 13413, and the liquid outlet flow path 13415 connected to the second heating container 13422 includes a first liquid outlet flow path 13416 and a third liquid outlet flow path 13418. The first liquid outlet flow path 13416 is connected to the blood return flow path 13406, and the third liquid outlet flow path 13418 is connected to the blood drawing flow path 13404 or the blood pumping flow path 13405.

Thus, in actual use, the replacement fluid in the second heating container 13422 can selectively flow into the blood return flow path 13406, the blood drawing flow path 13404 or the blood pumping flow path 13405.

Exemplarily, the second pipeline switching device 29 of the blood purification device 200 can control the first liquid outlet flow path 13416 to be connected and the third liquid outlet flow path 13418 to be disconnected, so that the replacement fluid in the replacement fluid supply container flows through the fifth liquid inlet flow path 13412, the replacement fluid pump pipeline section 1314, the sixth liquid inlet flow path 13413, the second heating container 13422 and the first liquid outlet flow path 13416 in sequence, and then flows to the blood return flow path 13406, thereby realizing the injection of replacement fluid into the blood filtered by the filter 300.

Alternatively, the second pipeline switching device 29 of the blood purification device 200 can control the first liquid outlet flow path 13416 to be disconnected and the third liquid outlet flow path 13418 to be connected, so that the replacement fluid in the replacement fluid supply container flows through the fifth liquid inlet flow path 13412, the replacement fluid pump pipeline section 1314, the sixth liquid inlet flow path 13413, the second heating container 13422 and the third liquid outlet flow path 13418 in sequence, and then flows to the blood drawing flow path 13404 or the blood pumping flow path 13405, thereby realizing the injection of replacement fluid into the blood not filtered by the filter 300.

In some embodiments, both the power panel 11 and the monitoring panel 12 are arranged separately from the heating panel 14, so that the heating panel 14 can be detached and mounted separately relative to the power panel 11, and the heating panel 14 can be detached and mounted separately relative to the monitoring panel 12.

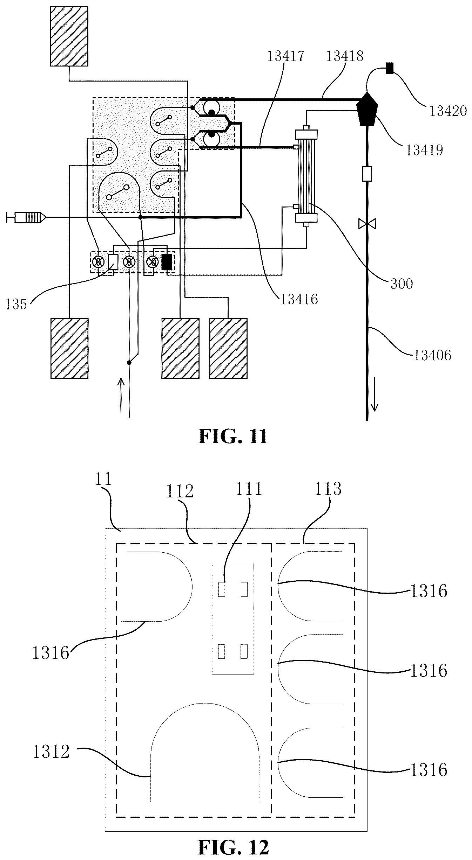

Please continue to refer to Fig. 11, in some embodiments, the output of the first liquid outlet flow path 13416 may be connected to the blood drawing flow path 13404 or the blood pumping flow path 13405, the output of the second liquid outlet flow path 13417 is configured to be connected to the medical liquid input of the filter 300, and the output of the third liquid outlet flow path 13418 is connected to the blood return flow path 13406.

Thus, the replacement fluid in the replacement fluid supply container can be selectively injected into the blood drawing flow path 13404 or the blood pumping flow path 13405 through the first liquid outlet flow path 13416, or injected into the blood return flow path 13406 through the third liquid outlet flow path. In addition, the input of the third liquid inlet flow path 13410 can be selectively connected to a dialysate supply container and injected into the filter 300 through the second liquid outlet flow path 13417, or the input of the third liquid inlet flow path 13410 can be selectively connected to a replacement fluid supply container and injected into the blood drawing flow path 13404 or the blood pumping flow path 13405 through the first liquid outlet flow path 13416.

In some embodiments, the panel kit 100 may also be provided without the heating panel 14 and the heating container 13414. For example, the output of the fourth liquid inlet flow path 13411 is connected to the input of the first liquid outlet flow path 13416 and the input of the second liquid outlet flow path 13417 respectively through a three-way joint or the like, and the output of the sixth liquid inlet flow path 13413 is connected to the input of the first liquid outlet flow path 13416 and the input of the third liquid outlet flow path 13418 respectively through a three-way joint or the like.

In this case, the input of the first liquid outlet flow path 13416, the input of the second liquid outlet flow path 13417 and the input of the third liquid outlet flow path 13418 may be mounted on the power panel 11.

In some embodiments, the blood return flow path 13406 is provided with a degassing pot 13419 and a degassing pot monitoring element 13420, the degassing pot monitoring element 13420 is connected to the degassing pot 13419. When the degassing pot monitoring element 13420 is connected to the degassing pot monitoring interface on the blood purification device 200, the degassing pot monitoring element 13420 is used for the blood purification device 200 to monitor the pressure inside the degassing pot 13419. For example, the degassing pot monitoring element 13420 may include a Luer connector.

In some embodiments, the pipeline assembly 13 further includes an electrostatic discharge element 135, which is configured to cooperate with the blood purification device 200 to discharge static electricity in the pipeline assembly 13. It can be understood that static electricity may also be generated when the pump assembly 22 drives the pump pipeline section 131 to move, so discharging the static electricity through the electrostatic discharge element 135 can avoid interference with other equipment used at the same time, such as an electrocardiograph monitor.

In some embodiments, at least one of the power panel 11 and the monitoring panel 12 is provided with the electrostatic discharge element 135. Thus, by integrating the electrostatic discharge element 135 into the corresponding power panel 11 and/or monitoring panel 12, the adaptation of the electrostatic discharge element 135 to the blood purification device 200 can be realized synchronously with the mounting of the corresponding power panel 11 and/or monitoring panel 12, thereby facilitating the mounting of the panel kit 100.

Of course, in some other embodiments, the electrostatic discharge element 135 may not be provided on the power panel 11 or the monitoring panel 12, which is not limited in the embodiments of the present disclosure.

In some embodiments, the blood purification device 200 is provided with a conductive member, and the electrostatic discharge element 135 is configured to connect to the conductive member to discharge static electricity. For example, the electrostatic discharge element 135 can form an electrical connection by contacting the conductive member, so as to be grounded through the conductive member.

In some embodiments, a part of the pipeline sections of the pipeline assembly 13 is made of a conductive material to form the electrostatic discharge element 135. Of course, the electrostatic discharge element 135 may also be a conductive element connected to the pipeline assembly 13, which is not limited in the embodiments of the present disclosure.

In some embodiments, the pump pipeline section 131 is arc-shaped. Thus, the arc-shaped structure enables the pump pipeline section 131 to be wound around the pump head of the pump assembly 22 more conveniently.

In some embodiments, the openings formed at both ends of the pump pipeline section 131 face the outer peripheral side of the power panel 11. Thus, with both ends of all pump pipeline sections 131 facing outward, the two ends of the pump pipeline section 131 can be conveniently connected to other corresponding flow paths in the panel kit 100.

Optionally, the openings formed at both ends of at least part of the pump pipeline sections 131 face the inner peripheral side of the power panel 11, which is not limited in the embodiments of the present disclosure.

Please continue to refer to Fig. 12, in some embodiments, the power panel 11 includes an adjacent first region 112 and second region 113. The first region 112 is located on one side of the second region 113 in a horizontal direction. The pump pipeline section 131 includes one blood pump pipeline section 1312 and a plurality of medical fluid pump pipeline sections 1316. If the blood pump pipeline section 1312 is adapted to the pump assembly 22, the blood pump pipeline section 1312 is configured to be driven by the pump assembly 22, so as to drive the blood to flow; if a medical fluid pump pipeline section 1316 is adapted to the pump assembly 22, the medical fluid pump pipeline section 1316 is configured to be driven by the pump assembly 22, so as to drive the medical liquid to flow. One of the medical fluid pump pipeline sections 1316 and the blood pump pipeline section 1312 are arranged in the first region 112 in a substantially vertical direction, and all the other medical fluid pump pipeline sections 1316 are arranged in the second region 113 in a substantially vertical direction. Thus, arranging the pump pipeline sections 131 in two columns makes full use of the space on the power panel 11.

The first region 112 being located on one side of the second region 113 in the horizontal direction may mean that the upper end of the first region 112 is flush with that of the second region 113, and the lower end of the first region 112 is flush with that of the second region 113.

Alternatively, the first region 112 being located on one side of the second region 113 in the horizontal direction may mean that the upper end of the first region 112 is higher than that of the second region 113. In this case, the lower end of the first region 112 may be lower than that of the second region 113, or higher than that of the second region 113, or flush with that of the second region 113.

The first region 112 being located on one side of the second region 113 in the horizontal direction may also mean that the upper end of the first region 112 is lower than that of the second region 113. In this case, the lower end of the first region 112 may be lower than that of the second region 113, or higher than that of the second region 113, or flush with that of the second region 113.

That is to say, in the embodiments of the present disclosure, the first region 112 being located on one side of the second region 113 in the horizontal direction does not necessarily mean the upper end of the first region 112 is flush with that of the second region 113 and the lower end of the first region 112 is flush with that of the second region 113.

In some embodiments, the first region 112 being located on one side of the second region 113 in the horizontal direction may mean that at least half of the region of the first region 112 in the vertical direction is horizontally opposite to the second region 113, and/or at least half of the region of the second region 113 in the vertical direction is horizontally opposite to the first region 112.

In some embodiments, when the medical fluid pump pipeline sections 1316 are adapted to the pump assembly 22, one of the medical fluid pump pipeline section 1316 in the first region 112 and the medical fluid pump pipeline sections 1316 in the lowermost of the second region 113 is configured to be driven by the pump assembly 22, so as to drive the anticoagulant to flow, and the other one of the medical fluid pump pipeline section 1316 in the first region 112 and the medical fluid pump pipeline sections 1316 in the lowermost of the second region 113 is configured to be driven by the pump assembly 22, so as to drive medical fluid discharged from the filter 300 to flow. Alternatively, when the medical fluid pump pipeline sections 1316 are adapted to the pump assembly 22, one of the medical fluid pump pipeline section 1316 in the first region 112 and the medical fluid pump pipeline section 1316 in the middle of the second region 113 is configured to be driven by the pump assembly 22, so as to drive the anticoagulant to flow, and the other one of the medical fluid pump pipeline section 1316 in the first region 112 and the medical fluid pump pipeline section 1316 in the middle of the second region 113 is configured to be driven by the pump assembly 22, so as to drive medical fluid discharged from the filter 300 to flow.

For example, the medical fluid pump pipeline section 1316 driven by the pump assembly 22 to drive anticoagulant to flow is the front pump pipeline section 1315, and the medical fluid pump pipeline section 1316 driven by the pump assembly 22 to drive medical fluid discharged from the filter 300 to flow is the waste liquid pressure pump pipeline section 1311.

In this case, regardless of whether the waste liquid pressure pump pipeline section 1311 is the medical fluid pump pipeline section 1316 in the first region 112 or the medical fluid pump pipeline section 1316 in lowermost of the second region 113, if the waste liquid pressure pump pipeline section 1311 is damaged, the leaked medical waste liquid from the waste liquid pressure pump pipeline section 1311 will not easily contaminate other medical pump pipeline sections 131 above the second region 113.

In addition, when the medical fluid pump pipeline section 1316 in the first region 112 is located above the blood pump pipeline section 1312 in the vertical direction, regardless of whether the front pump pipeline section 1315 is the medical fluid pump pipeline section 1316 in the first region 112 or the medical fluid pump pipeline section 1316 in lowermost of the second region 113, the front pump pipeline section 1315 is adjacent to the blood pump pipeline section 1312. This shortens the distance between the blood drawing flow path 13404 or blood pumping flow path 13405 connected to the blood pump pipeline section 1312 and the second liquid inlet flow path 13409 connected to the front pump pipeline section 1315, thus allowing the second liquid inlet flow path 13409 to be made shorter.

In some embodiments, the number of medical fluid pump pipeline sections 1316 may be at least four. For example, the medical pump pipeline sections 131 include a blood pump pipeline section 1312, a front pump pipeline section 1315, a dialysate pump pipeline section 1313 and a replacement fluid pump pipeline section 1314.

It can be understood that the pump body of the pump assembly 22 of the device housing 21 for pumping blood may be larger than that for pumping medical fluid, so as to ensure that the pump body for pumping blood can draw blood from the blood vessel of the patient. Correspondingly, the radius of the blood pump pipeline section 1312 is also larger than that of the medical fluid pump pipeline section 1316. Thus, by arranging the blood pump pipeline section 131 and one medical fluid pump pipeline section 1316 in the first region 112 and at least three other medical fluid pump pipeline sections 1316 in the second region 113, the space adjacent to the first region 112 can be fully utilized to integrate at least three medical fluid pump pipeline sections 1316, making the structure of the power panel 11 more compact, and the structure of the pump assembly 22 on the corresponding device housing 21 more compact as well.

Exemplarily, the pump pipeline sections 131 in the first region 112 are the front pump pipeline section 1315 and the blood pump pipeline section 1312 in top-to-bottom order, and the pump pipeline sections 131 in the second region 113 are the dialysate pump pipeline section 1313, the replacement fluid pump pipeline section 1314 and the waste liquid pressure pump pipeline section 1311 in top-to-bottom order.

Alternatively, the pump pipeline sections 131 in the first region 112 are the front pump pipeline section 1315 and the blood pump pipeline section 1312 in top-to-bottom order, and the pump pipeline sections 131 in the second region 113 are the dialysate pump pipeline section 1313, the waste liquid pressure pump pipeline section 1311 and the replacement fluid pump pipeline section 1314 in top-to-bottom order.

Alternatively, the pump pipeline sections 131 in the first region 112 are the front pump pipeline section 1315 and the blood pump pipeline section 1312 in top-to-bottom order, and the pump pipeline sections 131 in the second region 113 are the replacement fluid pump pipeline section 1314, the dialysate pump pipeline section 1313 and the waste liquid pressure pump pipeline section 1311 in top-to-bottom order.

Alternatively, the pump pipeline sections 131 in the first region 112 are the front pump pipeline section 1315 and the blood pump pipeline section 1312 in top-to-bottom order, and the pump pipeline sections 131 in the second region 113 are the replacement fluid pump pipeline section 1314, the waste liquid pressure pump pipeline section 1311 and the dialysate pump pipeline section 1313 in top-to-bottom order.

Alternatively, the pump pipeline sections 131 in the first region 112 are the waste liquid pressure pump pipeline section 1311 and the blood pump pipeline section 1312 in top-to-bottom order, and the pump pipeline sections 131 in the second region 113 are the dialysate pump pipeline section 1313, the replacement fluid pump pipeline section 1314 and the front pump pipeline section 1315 in top-to-bottom order.

Alternatively, the pump pipeline sections 131 in the first region 112 are the waste liquid pressure pump pipeline section 1311 and the blood pump pipeline section 1312 in top-to-bottom order, and the pump pipeline sections 131 in the second region 113 are the dialysate pump pipeline section 1313, the front pump pipeline section 1315 and the replacement fluid pump pipeline section 1314 in top-to-bottom order.

Alternatively, the pump pipeline sections 131 in the first region 112 are the waste liquid pressure pump pipeline section 1311 and the blood pump pipeline section 1312 in top-to-bottom order, and the pump pipeline sections 131 in the second region 113 are the replacement fluid pump pipeline section 1314, the dialysate pump pipeline section 1313 and the front pump pipeline section 1315 in top-to-bottom order.

Alternatively, the pump pipeline sections 131 in the first region 112 are the waste liquid pressure pump pipeline section 1311 and the blood pump pipeline section 1312 in top-to-bottom order, and the pump pipeline sections 131 in the second region 113 are the replacement fluid pump pipeline section 1314, the front pump pipeline section 1315 and the dialysate pump pipeline section 1313 in top-to-bottom order.

In some embodiments, the first connecting part 111 of the power panel 11 may be located at least in the first region 112. For example, the medical fluid pump pipeline section 1316 in the first region 112 and at least part of the first connecting part 111 are arranged side by side in the horizontal direction and above the blood pump pipeline section 1312, thus making full use of the space above the blood pump pipeline section 1312.

In the above embodiments, each embodiment is described with a focus on different aspects, and the details not elaborated in one embodiment can be referred to the relevant descriptions of other embodiments.

The panel kit 100 for the blood purification device 200 provided by the embodiments of the present disclosure has been described in detail above. Specific examples are used herein to elaborate the principles and implementation modes of the present disclosure. The descriptions of the above embodiments are only intended to help understand the methods and core ideas of the present disclosure; at the same time, for those skilled in the art, there will be changes in the specific implementation modes and application scopes based on the ideas of the present disclosure. In summary, the content of this specification should not be construed as a limitation to the present disclosure.

## Claims

1. A panel kit for a blood purification device, **characterized in that**, the panel kit comprises:
a power panel (11) and a monitoring panel (12), wherein the power panel (11) and the monitoring panel (12) are independent of each other and are configured to be detachably mounted on a device housing (21) of the blood purification device separately; and
a pipeline assembly (13) configured to connect a filter (300) to a blood vessel of a patient and a medical liquid container respectively; wherein
the pipeline assembly (13) comprises at least two pump pipeline sections (131), the pump pipeline sections (131) are mounted on the power panel (11), and if the pump pipeline sections (131) are adapted to a pump assembly (22) of the blood purification device, the pump pipeline sections (131) are configured to be driven by the pump assembly (22), so as to drive a blood or a medical liquid to flow; and
the pipeline assembly (13) further comprises at least two monitoring pipeline sections (132), the monitoring pipeline sections (132) are mounted on the monitoring panel (12), the monitoring pipeline sections (132) are configured to monitor parameter information of the blood and/or the medical liquid, the parameter information at least comprises a pressure parameter, at least one of the monitoring pipeline sections (132) is provided with a first interface (1321), and if the first interface (1321) is connected to a second interface (23) of the blood purification device, the monitoring pipeline section (132) is used for the blood purification device to monitor the parameter information; and the first interface (1321) is fixedly arranged relative to the monitoring panel (12) during a process of mounting the monitoring panel (12) to the device housing (21).

2. A panel kit for a blood purification device, **characterized in that**, the panel kit comprises:
a power panel (11) and a monitoring panel (12), wherein the power panel (11) and the monitoring panel (12) are independent of each other and are configured to be detachably mounted on a device housing (21) of the blood purification device separately; and
a pipeline assembly (13) configured to connect a filter (300) to a blood vessel of a patient and a medical liquid container respectively; wherein
the pipeline assembly (13) comprises at least two pump pipeline sections (131), the pump pipeline sections (131) are mounted on the power panel (11), and if the pump pipeline sections (131) are adapted to a pump assembly (22) of the blood purification device, the pump pipeline sections (131) are configured to be driven by the pump assembly (22), so as to drive a blood or a medical liquid to flow; and
the pipeline assembly (13) further comprises at least two monitoring pipeline sections (132), the monitoring pipeline sections (132) are mounted on the monitoring panel (12), the monitoring pipeline sections (132) are configured to monitor parameter information of the blood and/or the medical liquid, the parameter information at least comprises a pressure parameter, at least one of the monitoring pipeline sections (132) is provided with a first interface (1321), and if the first interface (1321) is connected to a second interface (23) of the blood purification device, the monitoring pipeline section (132) is used for the blood purification device to monitor the parameter information; and the first interface (1321) is movably arranged relative to the monitoring panel (12), and the movable arrangement allows a user to operate the first interface (1321), so as to achieve the connection between the first interface (1321) and the second interface (23).

3. The panel kit according to claim 1 or 2, wherein the first interface (1321) is fixedly connected with the corresponding monitoring pipeline section (132), or the first interface (1321) is connected to the monitoring pipeline section (132) through a connector; and
wherein the monitoring pipeline section (132) is detachably or fixedly connected with the monitoring panel (12).

4. The panel kit according to claim 1 or 2, wherein at least two of the monitoring pipeline sections (132) are provided with the first interface (1321), and if the first interface (1321) is connected to the second interface (23), the monitoring pipeline sections (132) are used for the blood purification device to monitor the pressure parameter; and/or,
if the first interface (1321) is connected to the second interface (23), the monitoring pipeline section (132) is used for the blood purification device to monitor the pressure parameter, and the at least two monitoring pipeline sections (132) further comprise a blood leakage monitoring pipeline section (1322), and if the blood leakage monitoring pipeline section (1322) is adapted to a blood leakage monitoring module on the device housing (21), the blood leakage monitoring pipeline section (1322) is used for the blood leakage monitoring module to monitor whether the blood on one side of a filter membrane of the filter (300) permeates to the other side.

5. The panel kit according to claim 1 or 2, wherein the monitoring pipeline section (132) further comprises a secondary membrane pressure monitoring pipeline section (1323), the secondary membrane pressure monitoring pipeline section (1323) is configured to connect a blood output of one filter (300) to a blood input of another filter (300), and the secondary membrane pressure monitoring pipeline section (1323) is configured to measure the pressure parameter of the blood.

6. The panel kit according to claim 1 or 2, wherein each of the pump pipeline sections (131) comprises a waste liquid pressure pump pipeline section (1311);
the pipeline assembly (13) further comprises a waste liquid flow path (13402), an output of the waste liquid flow path (13402) is connected to an input of the waste liquid pressure pump pipeline section (1311), and an input of the waste liquid flow path (13402) is configured to be connected to a medical liquid output of the filter (300);
the monitoring pipeline section (132) comprises a waste liquid pressure monitoring pipeline section (1324), the waste liquid pressure monitoring pipeline section (1324) is arranged on the waste liquid flow path (13402), and the waste liquid pressure monitoring pipeline section (1324) is used for measuring the pressure parameter of the medical liquid,
preferably, the pipeline assembly (13) further comprises a collection flow path (13403), an input of the collection flow path (13403) is connected to an output of the waste liquid pressure pump pipeline section (1311), and an output of the collection flow path (13403) is configured to be connected to a medical liquid collection container or a secondary membrane pressure monitoring pipeline section (1323); and
the monitoring pipeline section (132) further comprises a blood leakage monitoring pipeline section (1322), the blood leakage monitoring pipeline section (1322) is arranged on the waste liquid flow path (13402) or the collection flow path (13403), and if the blood leakage monitoring pipeline section (1322) is adapted to a blood leakage monitoring module on the device housing (21), the blood leakage monitoring pipeline section (1322) is used for the blood leakage monitoring module to monitor whether the blood on one side of a filter membrane of the filter (300) permeates to the other side.

7. The panel kit according to claim 1 or 2, wherein each of the pump pipeline sections (131) comprises a blood pump pipeline section (1312), and the pipeline assembly (13) further comprises:
a blood drawing flow path (13404), an input of the blood drawing flow path (13404) is configured to be connected to a blood drawing site of the blood vessel of the patient, and an output of the blood drawing flow path (13404) is connected to an input of the blood pump pipeline section (1312);
a blood pumping flow path (13405), an input of the blood pumping flow path (13405) is connected to an output of the blood pump pipeline section (1312), and an output of the blood pumping flow path (13405) is configured to be connected to a blood input of the filter (300); and
a blood return flow path (13406), an input of the blood return flow path (13406) is configured to be connected to a blood output of the filter (300), and an output of the blood return flow path (13406) is configured to be connected to a blood return site of the blood vessel of the patient,
preferably, the monitoring pipeline section (132) comprises a blood drawing pressure monitoring pipeline section (1325), the blood drawing pressure monitoring pipeline section (1325) is arranged on the blood drawing flow path (13404), and the blood drawing pressure monitoring pipeline section (1325) is configured to monitor the pressure parameter of the blood; and/or,
the monitoring pipeline section (132) further comprises a pressure monitoring pipeline section (1326) before filtering, the pressure monitoring pipeline section (1326) before filtering is arranged on the blood pumping flow path (13405), and the pressure monitoring pipeline section before filtering (1326) is configured to monitor the pressure parameter of the blood.

8. The panel kit according to claim 7, wherein the pipeline assembly (13) further comprises an injection flow path (13407), an input of the injection flow path (13407) is configured to be connected to an anticoagulant injection pump, and an output of the injection flow path (13407) is connected to the blood drawing flow path (13404) or the blood pumping flow path (13405); and/or,
each of the pump pipeline section (131) further comprises a front pump pipeline section (1315) before blood pump, the pipeline assembly (13) comprises a first liquid inlet flow path (13408) and a second liquid inlet flow path (13409), the first liquid inlet flow path (13408), the front pump pipeline section (1315) and the second liquid inlet flow path (13409) are connected in sequence, an input of the first liquid inlet flow path (13408) is configured to be connected to at least an anticoagulant supply container, and an output of the second liquid inlet flow path (13409) is connected to the blood drawing flow path (13404), the blood pumping flow path (13405) or the blood return flow path (13406).

9. The panel kit according to claim 7, wherein each of the pump pipeline sections (131) comprises a dialysate pump pipeline section (1313), the pipeline assembly (13) further comprises a third liquid inlet flow path (13410) and a fourth liquid inlet flow path (13411), the third liquid inlet flow path (13410), the dialysate pump pipeline section (1313) and the fourth liquid inlet flow path (13411) are connected in sequence, an input of the third liquid inlet flow path (13410) is configured to be connected to a dialysate supply container, and an output of the fourth liquid inlet flow path (13411) is configured to be connected to a medical liquid input of the filter (300); and/or,
each of the pump pipeline sections (131) further comprises a replacement fluid pump pipeline section (1314), the pipeline assembly (13) further comprises a fifth liquid inlet flow path (13412) and a sixth liquid inlet flow path (13413), the fifth liquid inlet flow path (13412), the replacement fluid pump pipeline section (1314) and the sixth liquid inlet flow path (13413) are connected in sequence, an input of the fifth liquid inlet flow path (13412) is configured to be connected to a replacement fluid supply container, and an output of the sixth liquid inlet flow path (13413) is connected to the blood drawing flow path (13404), the blood pumping flow path (13405) or the blood return flow path (13406).

10. The panel kit according to claim 9, wherein the panel kit further comprises a heating panel (14), and the heating panel (14) is configured to be detachably mounted on a heating region of the device housing (21);
the pipeline assembly (13) comprises a heating container (13414) and a liquid outlet flow path (13415), the liquid outlet flow path (13415) is connected to an output of the heating container (13414), and the heating container (13414) is mounted on the heating panel (14), so as to be mounted on the heating region through the heating panel (14),
preferably, each of the pump pipeline sections (131) comprises the dialysate pump pipeline section (1313), the heating container (13414) comprises a first heating container (13421), an input of the first heating container (13421) is connected to an output of the fourth liquid inlet flow path (13411), the liquid outlet flow path (13415) connected to the first heating container (13421) comprises a first liquid outlet flow path (13416) and a second liquid outlet flow path (13417), the first liquid outlet flow path (13416) is connected to the blood return flow path (13406), and the second liquid outlet flow path (13417) is configured to be connected to a medical liquid input of the filter (300); and/or,
each of the pump pipeline section (131) comprises the replacement fluid pump pipeline section (1314), the heating container (13414) comprises a second heating container (13422), an input of the second heating container (13422) is connected to an output of the sixth liquid inlet flow path (13413), the liquid outlet flow path (13415) connected to the second heating container (13422) comprises a first liquid outlet flow path (13416) and a third liquid outlet flow path (13418), the first liquid outlet flow path (13416) is connected to the blood return flow path (13406), and the third liquid outlet flow path (13418) is connected to the blood drawing flow path (13404) or the blood pumping flow path (13405).

11. The panel kit according to claim 7, wherein the blood return flow path (13406) is provided with a degassing pot (13419) and a degassing pot monitoring element (13420), the degassing pot monitoring element (13420) is connected to the degassing pot (13419), and if the degassing pot monitoring element (13420) is connected to a degassing pot monitoring interface on the device housing (21), the degassing pot monitoring element (13420) is used for the blood purification device to monitor a pressure inside the degassing pot (13419).

12. The panel kit according to claim 1 or 2, wherein a pipeline connected between each of the pump pipeline sections (131) and the monitoring pipeline section (132) is a flexible pipeline.

13. The panel kit according to claim 1 or 2, wherein each of the pump pipeline sections (131) is arc-shaped, and openings formed at two ends of each of the pump pipeline sections (131) face an outer peripheral side of the power panel (11), or openings formed at two ends of at least one of the pump pipeline sections (131) face an inner peripheral side of the power panel (11).

14. The panel kit according to claim 1 or 2, wherein the power panel (11) comprises a first region (112) and a second region (113) which are adjacent to each other, and the first region (112) is located on one side of the second region (113) in a horizontal direction;
each of the pump pipeline sections (131) comprises one blood pump pipeline section (1312) and a plurality of medical liquid pump pipeline sections (1316), if the blood pump pipeline section (1312) is adapted to the pump assembly (22), the blood pump pipeline section (1312) is configured to be driven by the pump assembly (22), so as to drive the blood to flow, and if the medical liquid pump pipeline sections (1316) are adapted to the pump assembly (22), the medical liquid pump pipeline sections (1316) are configured to be driven by the pump assembly (22), so as to drive the medical liquid to flow;
one of the medical liquid pump pipeline sections (1316) and the blood pump pipeline section (1312) are arranged in the first region (112) in a substantially vertical direction, and other medical liquid pump pipeline sections (1316) are arranged in the second region (113) in a substantially vertical direction,
preferably, if the medical liquid pump pipeline sections (1316) are adapted to the pump assembly (22), one of the medical liquid pump pipeline section (1316) in the first region (112) and a medical liquid pump pipeline section (1316) at a lowermost side of the second region (113) is configured to be driven by the pump assembly (22), so as to drive an anticoagulant to flow, and the other of the medical liquid pump pipeline section (1316) in the first region (112) and the medical liquid pump pipeline section (1316) at the lowermost side of the second region (113) is configured to be driven by the pump assembly (22), so as to drive the medical liquid flowing out of the filter (300) to flow; or,
if the medical liquid pump pipeline sections (1316) are adapted to the pump assembly (22), one of the medical liquid pump pipeline section (1316) in the first region (112) and a medical liquid pump pipeline section (1316) in the middle of the second region (113) is configured to be driven by the pump assembly (22), so as to drive an anticoagulant to flow, and the other of the medical liquid pump pipeline section (1316) in the first region (112) and the medical liquid pump pipeline section (1316) in the middle of the second region (113) is configured to be driven by the pump assembly (22), so as to drive the medical liquid flowing out of the filter (300) to flow.

15. The panel kit according to claim 1 or 2, wherein the panel kit is capable of being manually mounted, so as to mount the monitoring panel (12) at a second target position of the device housing (21) and synchronously enable connection between the second interface (23) and the first interface (1321); and if the first interface (1321) is connected to the second interface (23), the monitoring pipeline section (132) is used for the blood purification device to monitor the parameter information; or,
if the monitoring panel (12) is mounted at the second target position of the device housing (21), the second interface (23) can be extended to be connected to the first interface (1321); and if the first interface (1321) is connected to the second interface (23), the monitoring pipeline section (132) is used for the blood purification device to monitor the parameter information; or,
if the monitoring panel (12) is mounted at a second preset position of the device housing (21), the blood purification device is capable of driving the monitoring panel (12) to move to a second target position so as to enable connection between the second interface (23) and the first interface (1321); and if the first interface (1321) is connected to the second interface (23), the monitoring pipeline section (132) is used for the blood purification device to monitor the parameter information; or,
when the first interface (1321) is movably arranged relative to the monitoring panel (12), if the monitoring panel (12) is mounted at the second target position of the device housing (21), the first interface (1321) is capable of realizing connection between the first interface (1321) and the second interface (23) through manual operation; and if the first interface (1321) is connected to the second interface (23), the monitoring pipeline section (132) is used for the blood purification device to monitor the parameter information.
